# EUROPEAN PATENT SPECIFICATION

(11) **EP 0 516 099 B1**
(45) Date of publication and mention of the grant of the patent: **30.10.1996**
(21) Application number: 92108975.1
(22) Date of filing: 27.05.1992
(51) Int. Cl.: C07C 67/29, C07C 69/30, C07C 69/52

(54) **Use of polyoxyalkylene glycerol ether fatty acid esters as deinking agent fur waste paper generation.**
Verwendung von Polyoxyalkylenglycerolätherfettsäureestern als Tinteentferner für Altpapierverwertung.
Utilisation d'esters d'acides gras et du polyoxyalkylène glycérol éther comme agent de désencrage dans la récupératiom de vieux papiers.

(30) Priority: 30.05.1991 JP 155104/91; 31.05.1991 JP 157595/91
(43) Date of publication of application: 02.12.1992
(73) Proprietor: DAI-ICHI KOGYO SEIYAKU CO., LTD., Shimogyo-ku Kyoto (JP)
(72) Inventor: Nishizaki, Shoichi, Shiga (JP); Kitagawa, Toru, Nishikyo-ku, Kyoto (JP)
(74) Representative: Strehl Schübel-Hopf Groening & Partner

(56) References cited:
- EP-A- 0 335 295
- FR-A- 1 460 530
- US-A- 2 678 935

## Description

### BACKGROUND OF THE INVENTION

The present invention relates to the use of polyoxyalkylene glycerol ether fatty acid esters as a deinking agent for waste paper regeneration.

The deinking agents used today in the regeneration of waste printed paper such as newspaper, magazine paper, handbill paper, information recording paper, simili paper, etc., reflect many improvements made in deinking performance to cope with the increasing diversification of waste paper. For example, particularly in recent years, the shortage and consequent rising prices of pulp resources have made it essential to utilize waste paper to the best advantage. Meanwhile, recent advances in the printing technology and the consequent changes in the composition of ink used have made deinking a more and more sophisticated operation.

Under the circumstances, many improvements have also been made in equipment to promote deinking of waste paper but deinking relying on the use of chemicals is a dominant approach today. As such chemicals, a variety of compositions each comprising a deinking agent and one or more auxiliary agents such as an alkali, e.g. sodium hydroxide, sodium silicate, sodium carbonate, sodium phosphate, etc., a bleaching agent, e.g. hydrogen peroxide, hyposulfites, hypochlorites, etc., a sequestering agent, e.g. EDTA, DTPA, etc., have been employed. As to the deinking agent, there have been employed anionic surfactants such as alkylbenzene-sulfonic acid sodium salts, higher alcohol sulfate sodium salts, dialkyl sulfosuccinate sodium salts, ethoxylated higher alcohol sulfate ammonium salts, fatty acid sodium salts, etc. and nonionic surfactants such as adducts of ethylene oxide-propylene oxide or ethylene oxide to starting materials such as higher alcohols, fatty acids, alkylphenols, etc., as used singly or in combination.

However, among these conventional deinking compositions for regeneration of waste paper, the alkylbenzenesulfonates, higher alcohol sulfate ester salts and higher alcohol or alkylphenol-ethylene oxide adducts, which are proposed by Japanese Kokai Patent Publication No. 51-84905, and dialkyl sulfosuccinates, which are described in Japanese Patent Publication No. 56-17476, insure satisfactory ink dispersibility but are deficient in the ability to trap the stripped ink, with the result that the regenerated pulp is not adequate in brightness.

The fatty acid sodium salts additionally used in Japanese Patent Publication No. 51-13762 and No. 61-1556 are claimed to enhance the degree of elimination of stripped ink in the floatation process and thereby contribute to an increased brightness of regenerated pulp but the use of them alone or with hard water results in decreases in deinking effect.

Aside from the foregoing, Japanese Kokai Patent Publication No. 55-51891 discloses random adducts of propylene oxide and ethylene oxide to higher alcohols and Japanese Kokai Patent Publication No. 58-109696 discloses random adducts of propylene oxide and ethylene oxide to higher fatty acids. These chemicals are all used in the deinking of waste newspaper but because they are deficient in the ability to strip ink and eliminate the stripped ink, none afford a regenerated pulp with a sufficiently high degree of brightness. Japanese Patent publication No. 1-57194 discloses products of alkylene oxide addition to a mixture of a natural fat or oil and a polyhydric (at least trihydric) alcohol. Said products, which are alkylene oxide adducts to mono- and diesters formed by transesterification between the natural fat or oil and the polyhydric alcohol, have less than 3 terminal hydrophobic groups in their molecular structure, the number of hydroxyl groups being generally 1 to 2. While they are used in the deinking of waste newspaper, they can show the characteristic features of the hydrophobic groups only to a reduced extent and are insufficient in the ability to strip ink and be adsorbed on the stripped ink, failing to afford a regenerated pulp with a high degree of brightness.

On the other hand, offset-printed paper and information recording paper have recently been claiming an increasing proportion of the waste paper stock. Since the waste paper stock including such waste paper contains thermosetting resin vehicles and toner inks, for instance, the conventional deinking agents do not insure sufficient deinking effects so that high quality regenerated paper cannot be obtained.

The present invention provides a deinking agent which is high in ink stripping performance and stripped ink elimination performance regardless of the type of waste printed paper stock and allows it to obtain a high-quality, high-brightness pulp.

The deinking agent provided by the present invention is a deinking agent for waste paper regeneration which comprises a polyoxyalkylene glycerol ether fatty acid ester derived from natural fats and oils.

The polyalkylene glycerol ether fatty acid ester may be obtained by a method which comprises reacting an alkylene oxide or oxides directly with a mixture of a natural fat or oil, water and a catalyst.

In this method, the reaction includes ester exchange and alkylene oxide addition which proceed competitively and simultaneously in the presence of a small amount of water and a catalyst as reaction initiators to give fat or oil-alkylene oxide adducts. More detailedly, in a mixed system comprising a natural fat or oil, a small amount of water and a catalyst, fatty acid residue transfer occurs in part of ester bonding in the fat or oil, and an alkylene oxide adds to the resulting natural fat or oil-derived hydroxyl group. Then, intramolecular transesterification goes on in the thus-produced alkylene oxide adducts to give a new fat or oil-derived hydroxyl group, to which an alkylene oxide adds. Since the reaction is carried out in a mixed system, the transesterification and alkylene oxide addition reactions further take place between the starting material fat or oil and the above reaction product and, via a continuous series of reactions, give a polyalkylene oxide adduct. The alkylene oxide addition to a natural fat or oil is repeated in that manner and, finally, a fat or oil-alkylene oxide adduct with a polyether chain inserted between the ester bonding in the fat or oil is obtained, without any remaining ester bond between any glycerol hydroxyl group and the fatty acid.

The above synthetic product obtained by the method of the present invention is characterized in that the ratio between the number of moles of glycerol residues and the number of moles of fatty acid residues (as determined by fatty acid assay by an extractive method following saponification) is always constant and is approximately equal to 1:3. Said product is thus rich in fat or oil-proper alkyl groups (hydrophobic groups) and therefore can show excellent surfactant characteristics that cannot be exhibited by the prior art one-step reaction products.

The natural fat or oil to be employed in said method includes, among others, a variety of vegetable oils such as coconut oil, palm oil, olive oil, soybean oil, rapeseed oil, linseed oil, sunflower oil, etc., animal oils such as lard, beef tallow, bone oil, animal fat, etc. and fish oil, and further includes the corresponding hardened and semihardened oils, and refined oils and recovered oils as obtained in the process of purification of such fats or oils.

Water is used in accordance with said method in the form of purified water, such as distilled water or deionized water, industrial water, or well water, for instance. The catalyst to be used is an alkaline substance, an alkali metal hydroxide, carbonate or organic acid salt, or the like, which is generally used in alkylene oxide addition reactions, and includes, among others, sodium methylate, potassium methylate, sodium hydroxide, potassium hydroxide, potassium carbonate, sodium acetate and potassium lactate.

The alkylene oxide to be used to prepare said polyalkylene glycerol ether fatty acid ester includes ethylene oxide, propylene oxide, butylene oxide and the like. Since, however, deinking agents must be water-soluble, the use of ethylene oxide is essential. The proportion of ethylene oxide is preferably 40 to 100% by weight on the total alkylene oxide basis. The alkylene oxide addition is conducted to an extent of 5 to 200 moles, preferably 10 to 150 moles, per mole of the natural fat or oil-water mixture. The mode of alkylene oxide addition may be blockwise or random.

The mole ratio between water and the natural fat or oil should preferably be selected on a case-by-case basis and can be adjusted within a broad range. From the production and physical properties viewpoints, it is generally advisable that the natural fat or oil-to-water mole ratio should be 1:0.01 to 1:3, preferably 1:0.05 to 1:2. When water is used in an amount of more than 3 moles per mole of the natural fat or oil, the byproducts polyoxyalkylene glycol and fatty acid-alkylene oxide adducts are produced in unfavorably increased amounts. To use water in an amount smaller than 0.01 mole will undesirably make the reaction difficult to proceed.

In the practice of the invention, the amount of alkylene oxide is not critical but may be varied as desired. The alkylene oxide species and the extent of addition thereof can be selected depending on the intended use of the reaction product. For instance, reaction products which are mainly ethylene oxide adducts and have a molecular weight of 1,000 to 5,000 (5 to 100 moles of alkylene oxide added) are suited for use as emulsifiers, dispersants, detergents, dyeing assistants and textile auxiliaries. Reaction products which are mainly propylene oxide adducts and have a molecular weight of 3,000 to 6,000 (40 to 100 moles of alkylene oxide added) are suitable as penetrants, antifoamers, lubricants and wetting agents. Reaction products which are ethylene oxide-propylene oxide-butylene oxide mixed adducts and have a molecular weight of 4,000 to 10,000 (60 to 200 moles of alkylene oxides added) are suitable as cosmetics ingredients, antifogging agents and flotation agents.

The reaction temperature to be employed in producing the deinking agent according to the above method is preferably within the range of 120 to 160°C although any other temperature range may be employed provided that the alkylene oxide addition reaction can progress at sufficiently high rates. The catalyst for the alkylene oxide addition reaction is preferably used in an amount of about 0.01 to 0.3% by weight based on the product and, in that amount, can satisfactorily increase the reactivity of alkylene oxides. It is more preferable to carry out the reaction at an elevated pressure up to 10 kg/cm²G than to carry out the reaction at normal pressures. This reaction also gives, in certain amounts, polyalkylene glycols and fatty acid-alkylene oxide adducts as byproducts. However, these byproducts will not impair the effects of the deinking agent.

The deinking process comprises a series of waste paper disintegration, high-concentration bleach, and floatation, and the deinking agent of the invention may be added portionwise at the respective steps or all in one step. The preferred level of addition of the deinking agent is 0.1 to 1.0 weight % based on the raw material waste paper.

The deinking agent exhibits its excellent performance even when used in combination with the known deinking auxiliary agents, e.g. alkalis such as sodium hydroxide, sodium silicate, sodium carbonate, etc., bleaching agents such as hydrogen peroxide, sodium hypochlorite, etc. and decomposing enzymes such as lipase, cellulase and so on. Moreover, a satisfactory deinking effect may be achieved by using the deinking agent of the invention in combination with the known deinking agents such as anionic surfactants, e.g. alkylbenzenesulfonate sodium salts, higher alcohol sulfate salts, etc. and nonionic surfactants such as adducts of ethylene oxide to higher alcohols, fatty acids, alkylphenols, etc., the corresponding ethylene oxide-propylene oxide adducts, alkanolamides and ethylene oxide adducts thereof and so on.

The waste printed paper to which the deinking agent is applicable includes, among others, printed matter such as newspaper, magazine and book paper, OA reproduction paper, simili paper, handbill paper and so on, irrespective of the printing methods such as relief printing, offset printing, gravure printing and so on.

The deinking agent can be handled with ease since it has a particular composition in that it contains compounds having terminal alkyl groups as resulting from insertion of alkylene oxide chains at the ester bond sites of the natural fat or oil. From another aspect, it is a medium molecular weight substance comprising a particular molecular structure with ethylene oxide, propylene oxide, etc. added in a suitable combination. Therefore, when it is used in the disintegration stage of waste paper regeneration, even where the waste paper is offset-printed paper or information recording paper which does not lend itself well to stripping of ink, the hydrophobic groups in the deinking agent promote stripping of the ink from the pulp fiber and insure efficient dispersion in medium. Furthermore, since the deinking agent is low in foaming property, it permits a high degree of aeration and insures effective trapping of stripped ink in a floatator, enabling the industry to produce a high-quality regenerated pulp with improved brightness and a minimum of residual ink content.

When the polyalkylene glycerol ether fatty acid ester is used as a deinking agent according to the present invention, a high-brightness regenerated paper can be obtained regardless of kinds of waste printed paper. Furthermore, even if the waste paper stock contains those kinds of waste paper which can hardly be regenerated, or even in the face of variations in the printing methods used, a high-quality regenerated pulp can be invariably obtained. Therefore, the deinking agent contributes greatly to the progress of waste paper regeneration technology and industry.

### DETAILED DESCRIPTION OF PREFERRED EMBODIMENTS

The following examples are intended to illustrate the present invention in further detail and should by no means be construed as defining the scope of the invention. In the examples that follow, all percents (%) are by weight.

### Reference Example 1

A 5-liter autoclave was charged with 840 g (1 mole) of palm oil, 9 g (0.5 mole) of distilled water and 4 g of 100% KOH. The autoclave contents were heated to 145°C with stirring at a rate of 450 rpm and then 2,200 g (50 moles) of ethylene oxide was charged portionwise into the autoclave at a temperature of 130 to 150°C and a pressure of 2 kg/cm²G. After completion of the addition reaction of ethylene oxide, the reaction mixture was cooled to 80°C, and neutralized to pH 6 with glacial acetic acid, whereby a reaction product occurring as a pale-yellow viscous liquid was obtained in a yield of 99.2%. This product had a saponification value of 55.2 and a fatty acid moiety content of 24.7% as determined by the extraction method following saponification, the mole ratio of the fatty acid to each mole of the glycerol residue being 3. As for its physical properties, it was soluble in water, excellent in detergency, but low in foaming power, hence suitable as a raw material for detergent formulations. The above analytical data, the fact that it showed surface activity and the results of ester bond position analysis by NMR spectrometry indicated that said product was a polyoxyalkylene glycerol ether fatty acid ester with an alkylene oxide-derived polyether chain being inserted in each ester bond, namely between a hydroxyl group residue and a fatty acid residue, of the natural oil.

### Reference Example 2

A 5-liter autoclave was charged with 860 g (1 mole) of beef tallow and a solution of 6 g of NaOH in 18 g (1 mole) of well water. The autoclave contents were heated to 145°C with stirring at a rate of 450 rpm and then 2,320 g (40 moles) of propylene oxide was charged portionwise into the autoclave at a temperature of 125 to 140°C and a pressure of 3.5 kg/cm²G. After completion of the addition reaction of propylene oxide, the reaction mixture was cooled to 80°C, neutralized to pH 6 with hydrochloric acid and purified by desalting to give a pale-yellow liquid reaction product in 98.5% yield. This product had a saponification value of 52.6 and a fatty acid moiety content of 24.2% as determined by the extraction method following saponification, the mole ratio of the fatty acid to each mole of the glycerol residue being 3. As for its physical properties, it was insoluble in water, but was soluble in mineral oils and ethyl alcohol and gave solutions capable of promoting a sliding motion, hence said product was suited for use as a lubricant additive. When added to lubricating oils, this product successfully provided them with a detergent-dispersant property and improved their oiliness.

### Reference Example 3

A 5-liter autoclave was charged with 943 g (1 mole) of rapeseed oil, 36 g (2 moles) of industrial water and 8 g of 100% potassium carbonate. The autoclave contents were heated to 155°C with stirring at a rate of 450 rpm and then 880 g (20 moles) of ethylene oxide was introduced portionwise into the autoclave at a temperature of 130 to 150°C and a pressure of 2.0 kg/cm²G. After completion of the addition reaction of ethylene oxide, 2,030 g (35 moles) of propylene oxide and 288 g (4 moles) of butylene oxide were introduced into the autoclave for block addition thereof at a temperature of 120 to 140°C and a pressure of 3.0 kg/cm²G. Thereafter, the reaction mixture was cooled to 80°C and neutralized to pH 6.5 with glacial acetic acid, whereby a pale-yellow liquid reaction product was obtained in a yield of 99.7%. This product had a saponification value of 40.3 and a fatty acid moiety content of 20.5% as determined by the extraction method following saponification, the mole ratio of the fatty acid to each mole of the glycerol residue being 3. As for its physical properties, it was soluble in water and gave transparent aqueous solutions. At a concentration of 5%, the clouding point was 30°C. A 0.1% aqueous solution showed a surface tension of 40.7 dynes/cm (20°C) and was excellent in penetrating property. Furthermore, the product showed potent antifoaming property and accordingly was suited for use as an antifoamer.

### Reference Example 4

A 5-liter autoclave was charged with 1,735 g (2 moles) of hardened beef tallow and 4 g of 100% KOH and the autoclave contents were heated to 140°C with stirring at a rate of 450 rpm. Then, 9 g (0.5 mole) of deionized water was added and then 1,320 g (30 moles) of ethylene oxide was introduced portionwise into the autoclave at the same temperature and a pressure of 3.0 kg/cm²G. After completion of the addition reaction of ethylene oxide, the reaction mixture was cooled to 80°C and neutralized to pH 6.5 with glacial acetic acid to give a pale-yellow past-like reaction product in 99.0% yield. This product had a saponification value of 109.9 and a fatty acid content of 51.0% as determined by the extraction method following saponification, the mole ratio of the fatty acid to each mole of the glycerol residue being 3. As for its physical properties, it was soluble in water to give transparent solutions. At a concentration of 5%, the clouding point was 80.5°C, indicating its good water solubility. A 0.1% aqueous solution showed a surface tension of 37.8 dynes/cm (20°C). The product was excellent in emulsifying power and dispersibility and suited for use as an emulsifying agent in synthetic fiber lubricants or a soaping agent to be used after dyeing of polyester fibers with a disperse dye.

### Reference Example 5

Ethylene oxide addition was carried out under the same conditions as used in Example 1 except that 1,760 g (2 moles) of lard and 1.8 g (0.1 mole) of well water were used in lieu of palm oil and distilled water, respectively, and that ethylene oxide was used in an amount of 440 g (10 moles). After completion of the reaction, the reaction mixture was cooled to 80°C and neutralized to pH 6.5 with glacial acetic acid to give a pale-yellow liquid reaction product in 99.4% yield. This product had a saponification value of 152.9 and a fatty acid moiety content of 72.1% as determined by the extraction method following saponification, the mole ratio of the fatty acid to each mole of the glycerol residue being 3. As for its physical properties, it was emulsified and dispersed in water and at the same time suitable as an emulsifier. Furthermore, when used as a cold rolling oil, it showed emulsifier and lubricant properties stably for a long period of time. It prevented oil scum formation due to a fine iron powder produced in the rolling step as well as surface soiling of steel sheets and reduction rolls effectively.

### Reference Example 6

Random addition using a mixture of 1,740 g (30 moles) of propylene oxide and 1,760 g (40 moles) of ethylene oxide was carried out under the same conditions as used in Example 2 except that 640 g (1 mole) of refined coconut oil and a solution of 4 g of KOH in 9 g (0.5 mole) of industrial water were charged initially. After completion of the reaction, the reaction mixture was cooled to 80°C, neutralized to pH 6.5 with hydrochloric acid, and desalted for purification. A pale-yellow liquid reaction product was obtained in 98.6% yield. This product had a saponification value of 40.5 and a fatty acid moiety content of 13.3% as determined by the extraction method following saponification, the mole ratio of the fatty acid to each mole of the glycerol residue being 3. As for its physical properties, it was soluble in mineral oils, water and alcohols and gave transparent solutions. At a concentration of 5%, the clouding point was 74.0°C, and a 0.1% aqueous solution showed a surface tension of 39.5 dynes/cm (20°C). Furthermore, this product showed good detergency and rinsing property, which are required of cosmetics ingredients, hence it was suited for use as a shampoo ingredient.

### Example 1

The deinking agents (polyoxyalkylene glycerol ether fatty acid esters) of the present invention as shown in Table 1 were produced according to Reference Examples 1 to 6.

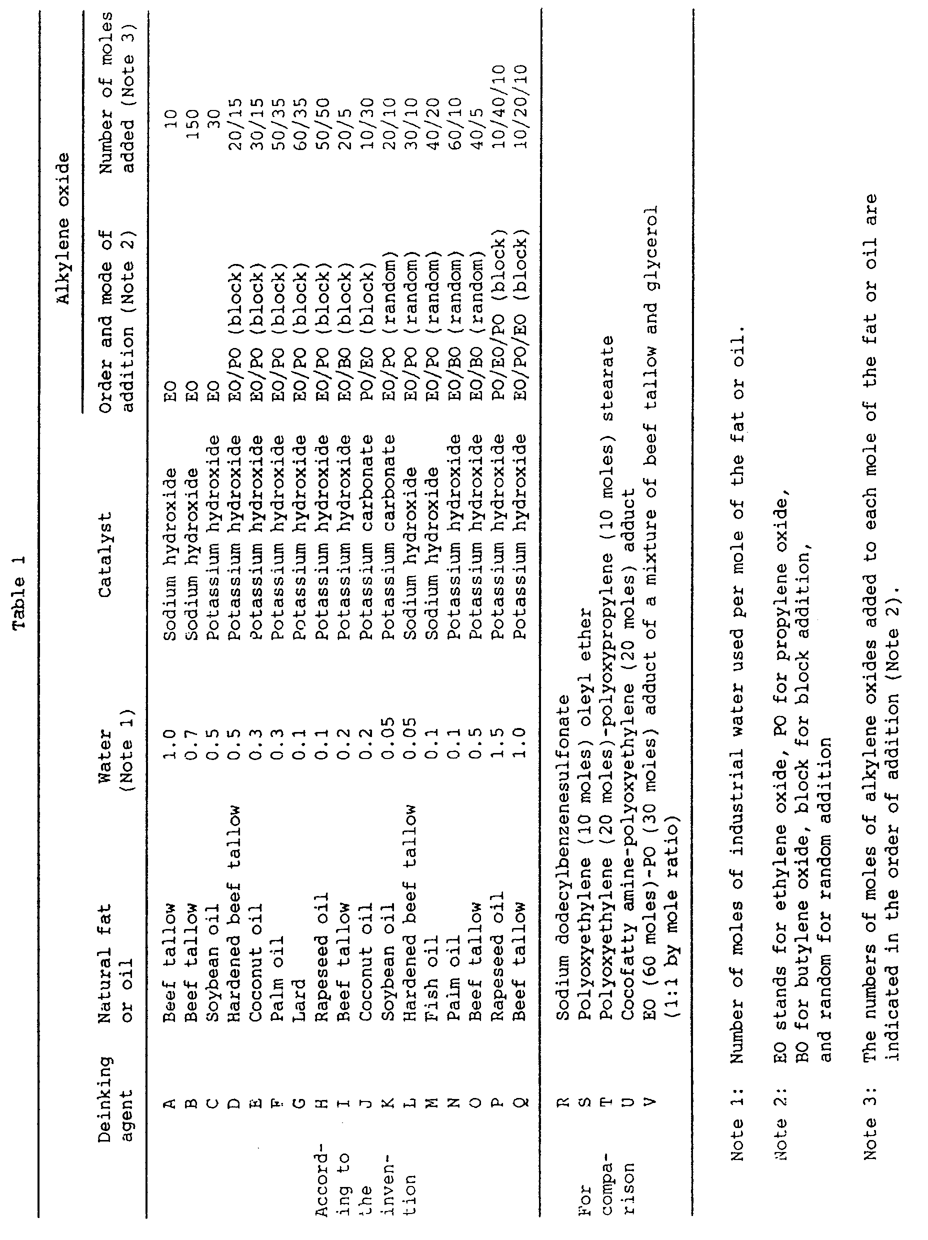

### Examples 2 through 18 and Comparative Examples 1 through 5

A pulp disintegrator (JIS P-8209) was charged with a slit waste paper stock consisting of 70% of newspaper (offset/relief = 8/2, all within 1-2 months after printing) and 30% of handbill paper and, based on the weight of waste paper, 1.5% of sodium hydroxide, 3% of 40% No. 3 sodium silicate, 3% of 30% aqueous solution of hydrogen peroxide and 0.3% of one of the deinking agents indicated in Table 1. The charge was diluted to a paper stock concentration of 5% with warm water and a disintegration treatment was carried out at about 50°C for 20 minutes.

The resulting pulp slurry was ripened at 50°C for 60 minutes and diluted with water to a pulp concentration of 1.0%. After 1.0% (based on waste paper) of CaCl₂ was added, floatation was carried out at 30°C for 10 minutes. After the floatation, the pulp slurry was concentrated to 6% and, then, diluted to 1% with water. The diluted slurry was adjusted to pH 5 and processed with a TAPPI standard sheet machine into a regenerated paper weighing 100 g/m².

This regenerated paper was determined for brightness with a multipurpose reflectometer (the Hunter brightness according to JIS P-8123). As to the ink residues in this regenerated paper, the residual ink count and the unstripped ink count were determined with an image processor (x 126). The results are set forth in Table 2.

**Table 2**

| | | Deinking agent | Regenerated paper after floatation | | |
|---|---|---|---|---|---|
| | | | Brightness (%) | Residual ink count (count/field) | Unstripped ink count (count/field) |
| Examples | 2 | A | 53.8 | 26 | 26 |
| | 3 | B | 54.0 | 20 | 24 |
| | 4 | C | 54.6 | 16 | 20 |
| | 5 | D | 55.0 | 12 | 17 |
| | 6 | E | 55.4 | 10 | 15 |
| | 7 | F | 54.8 | 14 | 18 |
| | 8 | G | 56.0 | 5 | 8 |
| | 9 | H | 55.6 | 8 | 14 |
| | 10 | I | 54.8 | 14 | 18 |
| | 11 | J | 55.2 | 11 | 16 |
| | 12 | K | 55.8 | 6 | 10 |
| | 13 | L | 55.6 | 8 | 13 |
| | 14 | M | 55.1 | 12 | 16 |
| | 15 | N | 55.4 | 10 | 15 |
| | 16 | O | 54.0 | 18 | 22 |
| | 17 | P | 53.2 | 28 | 30 |
| | 18 | Q | 53.6 | 26 | 28 |
| Comparative Examples | 1 | R | 49.0 | 76 | 68 |
| | 2 | S | 48.8 | 80 | 86 |
| | 3 | T | 49.6 | 62 | 58 |
| | 4 | U | 47.2 | 102 | 110 |
| | 5 | V | 52.0 | 38 | 42 |

### Examples 19 through 35 and Comparative Examples 6 through 10

A pulp disintegrator (JIS P-8209) was charged with a slit waste paper stock consisting of 40% of newspaper (offset/relief = 8/2, all within 1-2 months after printing), 20% of handbill paper, 20% of magazine paper and 20% of OA reproduction paper and, based on the weight of waste paper, 1.5% of sodium hydroxide, 3% of 40% No. 3 sodium silicate, 3% of 30% aqueous solution of hydrogen peroxide and 0.3% of one of the deinking agents shown in Table 1. The charge was diluted to a paper stock concentration of 5% with warm water and a disintegration treatment was carried out at about 50°C for 20 minutes. Thereafter, the same procedure as described in Examples 8 through 24 and Comparative Examples 6 through 10 was followed to give a regenerated paper weighing 100 g/m². For the thus-obtained regenerated paper, the brightness, residual ink count, and unstripped ink count were determined. The results are set forth in Table 3.

**Table 3**

| | | Deinking agent | Regenerated paper after floatation | | |
|---|---|---|---|---|---|
| | | | Brightness (%) | Residual ink count (count/field) | Unstripped ink count (count/field) |
| Examples | 19 | A | 53.6 | 25 | 23 |
| | 20 | B | 53.8 | 24 | 22 |
| | 21 | C | 54.8 | 17 | 18 |
| | 22 | D | 55.0 | 15 | 16 |
| | 23 | E | 55.5 | 10 | 12 |
| | 24 | F | 55.4 | 11 | 12 |
| | 25 | G | 56.2 | 7 | 6 |
| | 26 | H | 55.6 | 9 | 10 |
| | 27 | I | 55.8 | 8 | 9 |
| | 28 | J | 55.4 | 12 | 11 |
| | 29 | K | 55.8 | 8 | 10 |
| | 30 | L | 55.7 | 9 | 9 |
| | 31 | M | 55.3 | 13 | 12 |
| | 32 | N | 55.5 | 11 | 12 |
| | 33 | O | 54.2 | 20 | 21 |
| | 34 | P | 53.5 | 26 | 25 |
| | 35 | Q | 54.0 | 21 | 22 |
| Comparative Examples | 6 | R | 48.3 | 86 | 95 |
| | 7 | S | 48.0 | 88 | 86 |
| | 8 | T | 49.0 | 74 | 69 |
| | 9 | U | 47.6 | 98 | 105 |
| | 10 | V | 52.3 | 40 | 39 |

## Claims

1. The use of a polyoxyalkylene glycerol ether fatty acid ester derived from natural fats and oils as a deinking agent for waste paper regeneration.

2. The use according to claim 1, wherein the polyoxyalkylene glycerol ether fatty acid ester is the product of the addition of 5 to 200 mols of an ethylene oxide-containing alkylene oxide reactant to each mole of a natural fat or oil-water mixture.

3. The use according to claim 1 or 2, wherein the alkylene oxide reactant contains ethylene oxide in an amount of 40 to 100 % by weight.

## Patentansprüche

1. Verwendung eines Polyoxyalkylenglycerinether-fettsäureesters, der von natürlichen Fetten und Ölen abgeleitet ist, als Entfärbungsmittel für die Regenerierung von Abfallpapier.

2. Verwendung nach Anspruch 1, wobei der Polyoxyalkylenglycerinether-fettsäureester das Produkt der Addition von 5 bis 200 Mol eines Ethylenoxid enthaltenden Alkylenoxid-Reaktanten an jedes Mol eines natürlichen Fettes oder Öles in einem Gemisch mit Wasser ist.

3. Verwendung nach Anspruch 1 oder 2, wobei der Alkylenoxid-Reaktant Ethylenoxid in einem Anteil von 40 bis 100 Gew.-% enthält.

## Revendications

1. Utilisation d'un ester d'acide gras de polyoxyalkylèneglycéroléther provenant d'huiles et de graisses naturelles comme agent de désencrage pour la régénération de vieux papiers.

2. Utilisation selon la revendication 1, dans laquelle l'ester d'acide gras de polyoxyalkylèneglycéroléther est le produit de l'addition de 5 à 200 moles d'un réactif de type oxyde d'alkylène contenant de l'oxyde d'éthylène par mole d'un mélange huile ou graisse naturelle-eau.

3. Utilisation selon la revendication 1 ou 2, dans laquelle le réactif de type oxyde d'alkylène contient de l'oxyde d'éthylène en une quantité de 40 à 100% en poids.
